# EUROPEAN PATENT SPECIFICATION

(11) **EP 3 247 414 B1**
(45) Date of publication and mention of the grant of the patent: **21.11.2018**
(21) Application number: 16702008.0
(22) Date of filing: 22.01.2016
(51) Int. Cl.: A61L 27/36

(54) **DETOXIFICATION AND STABILIZATION OF IMPLANTABLE OR TRANSPLANTABLE BIOLOGICAL MATERIAL**
ENTGIFTUNG UND STABILISIERUNG VON IMPLANTIERBAREM ODER TRANSPLANTIERBAREM BIOLOGISCHEM MATERIAL
DÉTOXICATION ET STABILISATION DE MATÉRIAU BIOLOGIQUE IMPLANTABLE OU TRANSPLANTABLE

(30) Priority: 22.01.2015 ZA 201500478
(43) Date of publication of application: 29.11.2017
(73) Proprietor: University Of The Free State, Bloemfontein 9301 (ZA)
(72) Inventor: BESTER, Dreyer, 9301 Bloemfontein (ZA); SMIT, Francis Edwin, 9301 Bloemfontein (ZA); VAN DEN HEEVER, Johannes, 9301 Bloemfontein (ZA); BOTES, Lezelle, 9301 Bloemfontein (ZA); DOHMEN, Pascal Maria Chris Eugène, 14195 Berlin (DE)
(74) Representative: Huenges, Martin
(86) International application number: PCT/IB2016/050320
(87) International publication number: WO 2016/116895

(56) References cited:
- WO-A1-2011/057174
- WO-A2-2011/042794
- US-B2- 7 438 850

## Description

### BACKGROUND OF THE INVENTION

Tissue engineering, for example whole organ engineering, could help to address the problems discussed in the background to the invention, since the tissue used is biological and there is no rejection potential. Additionally, there is the potential of tissue regeneration and remodeling. In order to carry out tissue engineering the classical triangle is needed: a scaffold, a large number of different autologous cells and a bioreactor.

US 7438850 describes a four-step sterilization method for the production of implantable or transplantable biological material of animal or human origin which comprises 1) a treatment with an antibiotic, 2) a further treatment with deoxycholic acid, 3) a treatment with lipoprotein solution for removal of the deoxycholic acid followed by 4) a treatment with an alcohol.

It is an object of this invention to provide an improved method of detoxification and stabilization of implantable or transplantable biological material of human or animal origin.

### SUMMARY OF THE INVENTION

This invention relates to method of detoxification and stabilization of implantable or transplantable biological material of human or animal origin according to claim 1.

The antibiotic solution may contain at least one, preferably all of the following antibiotics:
Amphotericin B, in an amount of 5 - 100, typically5-15, preferably 10µg/ml,
Ciprofloxacin, in an amount of 5 - 200, typically 45 - 55, preferably 50µg/ml,
Cefuroxin, in an amount of 20 - 1500, typically 740-760, preferably 750µg/ml,
Penicillin, in an amount of 20 - 1000, typically 180 - 220, preferably 200µg/ml,
Streptomycin in an amount of 20 - 1000, typically 180 - 220, typically 200µg/ml.
Preferably, the antibiotic solution contains no Ca or Mg.

The solution to remove the secondary bile acid surfactant contains a lipopeptide antimicrobial agent such as Fengycin or Iturin A in an amount of 5-800, preferably 20µg/ml.
After step 4), the material is introduced to a storage solution containing antibiotics, preferably selected from one or all of the following antibiotics: Amphotericin B, typically in an amount of 5-15, preferably 10µg/ml, Penicillin, typically in an amount of 80 - 120, typically 100µg/ml, Streptomycin, typically in an amount of 80 - 120, typically 100µg/ml.

The invention also relates to the solution containing an secondary bile acid surfactant for use in a method of detoxification and stabilization of implantable or transplantable biological material of human or animal origin according to claim 10.

### BRIEF DESCRIPTION OF THE DRAWINGS

- **Figure 1**: is a light microscopy photograph of tissue from a heart valve wall treated with a deoxycholic acid solution;
- **Figure 2**: is a light microscopy photograph of tissue from a heart valve wall treated with a deoxycholic acid and sodium dodecyl sulfate solution;
- **Figure 3**: is a light microscopy photograph of tissue from a heart valve wall treated with a deoxycholic acid, sodium dodecyl sulfate and Triton-x100 solution of the invention;
- **Figure 4**: is a light microscopy photograph of tissue from a leaflet of a heart valve wall treated with a deoxycholic acid solution;
- **Figure 5**: is a light microscopy photograph of tissue from a leaflet of a heart valve wall treated with a deoxycholic acid and sodium dodecyl sulfate solution;
- **Figure 6**: is a light microscopy photograph of tissue from a leaflet of a heart valve wall treated with a deoxycholic acid, sodium dodecyl sulfate and Triton-x100 solution of the invention;
- **Figure 7**: is a light microscopy photograph of pericardial tissue treated with a deoxycholic acid solution;
- **Figure 8**: is a light microscopy photograph of pericardial tissue treated with a deoxycholic acid and sodium dodecyl sulfate solution;
- **Figure 9**: is a light microscopy photograph of pericardial tissue treated with a deoxycholic acid, sodium dodecyl sulfate and Triton-x100 solution of the invention; and
- **Figure 10**: is a light microscopy photograph of myocardium tissue treated with a deoxycholic acid, sodium dodecyl sulfate and Triton-x100 solution of the invention.

### DESCRIPTION OF PREFERRED EMBODIMENTS

In accordance with the method of the present invention, tissue of human or animal origin is treated in four successive steps:
1) treatment of the tissue with an antibiotic solution;
2) treatment of the tissue in a solution containing a secondary bile acid surfactant;
3) treatment of the tissue in a solution to remove the secondary bile acid surfactant; and
4) treatment of the tissue in a primary alcohol.

The solution of Step 2) is a physiological solution containing a secondary bile acid surfactant which contains a triple combination of:
- deoxycholic acid or ursodeoxycholic acid
- an anionic surfactant, preferably a sulfate such as sodium dodecyl sulfate, ammonium dodecyl sulfate or potassium lauryl sulfate, and
- a non-ionic surfactant.

Deoxycholic acid (DOA), is a secondary bile acid surfactant. Alternatively ursodeoxycholic acid can also be used. It also takes care of the lipid parts of the membrane, not only the protein part. Additionally it has an anti-inflammatory activity which is also important in the production of an extracellular matrix.
The preferred anionic surfactant is sodium dodecyl sulfate (SDS), alternative anionic surfactants are ammonium dodecyl sulfate and potassium lauryl sulfate, which are of the same group, however a little different. These are anionic surfactant (anorganic) or detergent surfactant which denaturate proteins, but also microbicide including enveloped and non-enveloped viruses will be destroyed.
The preferred non-ionic surfactant is Triton-x100 (a polyoxyethylene surfactant) which will not denaturate proteins but results in membrane distortion to prepare the tissue for sodium dodecyl sulfate and deoxycholic acid to destroy the tissue (denaturate).
This triple combination has a synergistic effect: i.e. the combination of all three components allows the concentration of the individual components to be reduced. A lower concentration of components means that, in use, there is less chance that the stability of the extracellular matrix/scaffold will be changed during the detoxification and stabilization of the scaffold.

After step 4), the tissue is rinsed to remove all debridement out of the extracellular matrix. Thereafter, the material is introduced to a storage solution containing antibiotics, preferably selected from one or all of the following antibiotics:
Amphotericin B in an amount of 5-15 µg/ml,
Penicillin in an amount of 80 - 120 µg/ml,
Streptomycin in an amount of 80 - 120 µg/ml.

The method may be used in the preparation of a heart, heart valves, grafts, patch material etc. and also other organs or tissue such as omentum.

The invention is described in more detail with reference to the following Examples. The invention is not restricted to these Examples.

### Examples

An Example of the invention is the preparation of a large size heart, which was decellularized to create a scaffold on which autologous cells can be transplanted and later on implanted.

Technique to modify tissue by detoxification and stabilization:

### Step 1) - treatment of the tissue with an antibiotic solution

The antibiotic solution without Ca or Mg contains a cocktail of antibiotics and antimycotic medication with flow or without flow at a shaker for several hours and at room temperature or at 37°C

| | |
|---|---|
| 10µg/ml | Amphotericin B |
| 50µg/ml | Ciprofloxacin |
| 750µg/ml | Cefuroxin |
| 200 U/ml | Penicillin |
| 200 µg/ml | Streptomycin |

Thereafter the tissue is treated with distilled or purified water also for a particular time 15 minutes to 1 hour at room temperature or 37°C.

### Step 2) - treatment of the tissue in a solution containing a secondary bile acid surfactant

The tissue is treated with a combination of deoxycholic acid (DOA) (0.5% v/v), sodium dodecyl sulfate (SDS) (2% v/v), and Triton x-100 (0.5% v/v). These substances have been used in the past, however never all together since there is a synergic effect in case using them together. Therefore the concentration can be lower and there is less chance that the stability of the extracellular matrix / scaffold can be changed. The collagen can be destroyed and deterioration can be increased due to this. This step is carried out at a particular temperature (room or 37°C) for several hours or days (long).

### Step 3) - treatment of the tissue in a solution to remove the the secondary bile acid surfactant

The tissue is treated with Fengycin 100 µg/ml in DMSO 5 mmol to sterilize and to remove the DOA/SDS and Triton out of the tissue. It is also possible to use Iturin A for several hours. These are lipopeptide antimicrobial agents that are also antifungal. It will stabilize the tissue more. Concentrations can be changed, depending on the time. Temperature can also be different (room temperature or 37°C is optimal).. A shaker or flow can be used.

### Step 4) - treatment of the tissue in a primary alcohol

Ethanol or another alcohol should be used to stabilize the tissue but will also sterilize the tissue. Again with or without shaker or under flow conditions for different time (several hours and at different temperature (room temperature or 37°C is optimal).

Extensive rising of the tissue to get all the debridement out of the extracellular matrix. It would also be possible to control this by measurement to minimize the debridement at a minimum on the end.

Final step is storage with a specific store solution:

| | |
|---|---|
| 10µg/ml | Amphotericin B |
| 100 µg/ml | Penicillin (reduced concentration) |
| 100 µg/ml | Streptomycin (reduced concentration) |

The process of the invention described above was carried out on different tissues and comparative tests were conducted using a single solution containing either deoxycholic acid, or sodium dodecyl sulfate, or Triton-X100, and a double solution containing either deoxycholic acid with sodium dodecyl sulfate, or deoxycholic acid with Triton-X100, or sodium dodecyl sulfate with Triton-X100. Only in the triple deoxycholic acid solution of the invention containing an organic acid surfactant bile acid (secondary); an anionic surfactant, and a non-ionic surfactant results in a cell free tissue without destroying the extracellular structures.

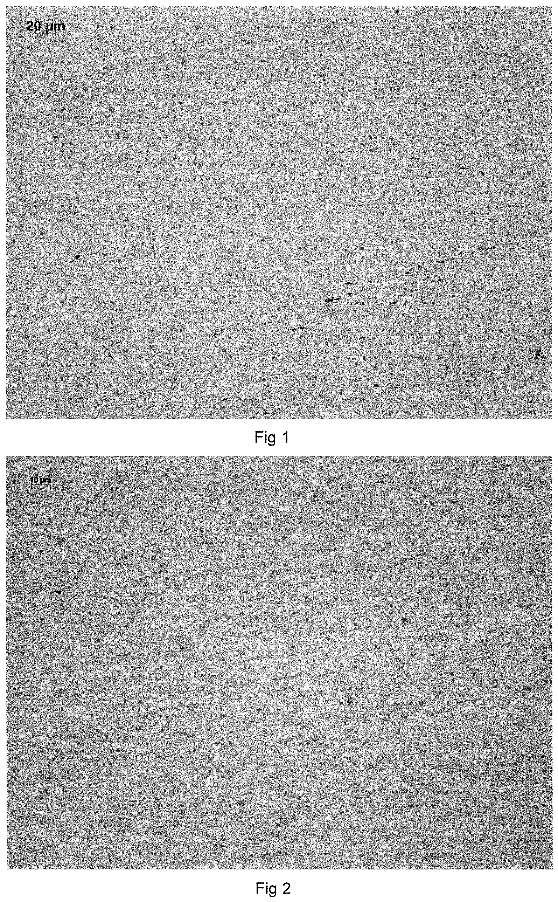

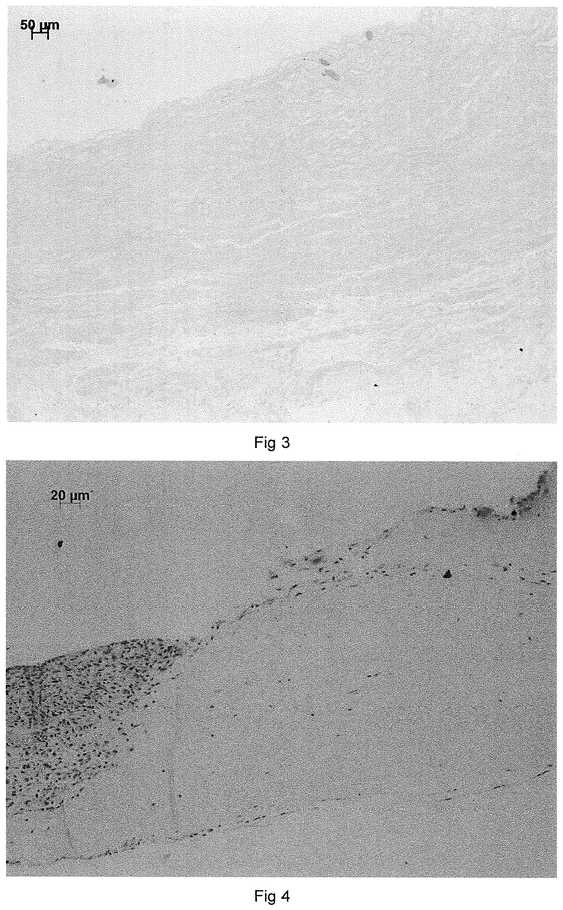

Figure 1 is a light microscopy photograph of tissue from a heart valve wall treated with the single deoxycholic acid solution. Figure 2 is a light microscopy photograph of tissue from a heart valve wall treated with the double deoxycholic acid solution. Figure 3 is a light microscopy photograph of tissue from a heart valve wall treated with the triple deoxycholic acid solution of the invention. From Figure 1, it can be seen that treatment with a single solution containing deoxycholic acid, one a large part of the tissue is free of cells, however not completely. Figure 2, which shows the result of treatment with the double deoxycholic acid solution shows an improvement, with additional reduction of cells in the tissue. However, as shown in Figure 3, it is only the triple deoxycholic acid solution of the invention where there are no cells available any longer, and thus achieves complete decellularization.

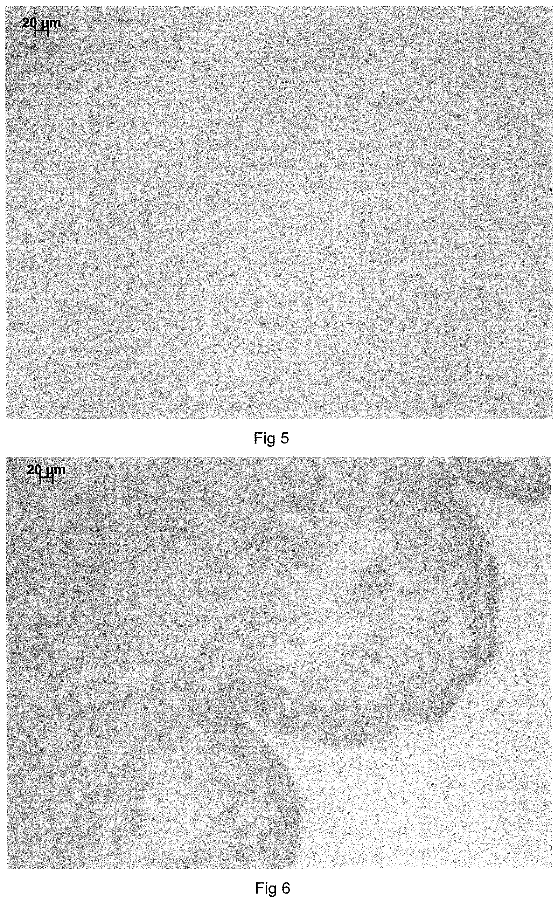

Figure 4 is a light microscopy photograph of tissue from a leaflet of a heart valve wall treated with the single deoxycholic acid solution. Figure 5 is a light microscopy photograph of tissue from a leaflet of a heart valve wall treated with the double deoxycholic acid solution. Figure 6 is a light microscopy photograph of tissue from a leaflet of a heart valve wall treated with the triple deoxycholic acid solution of the invention. From Figure 4, it can be seen that treatment with a single solution containing deoxycholic acid, one a large part of the tissue is free of cells, however not completely. Figure 5, which shows the result of treatment with the double deoxycholic acid solution shows an improvement, with additional reduction of cells in the tissue. However, as shown in Figure 6, it is only the triple deoxycholic acid solution of the invention where there are no cells available any longer, and thus achieves complete decellularization.

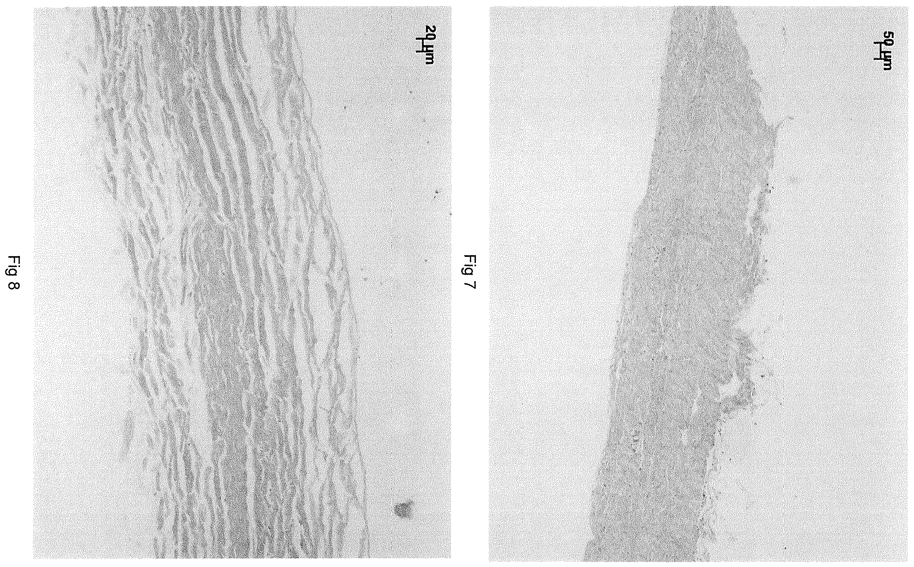

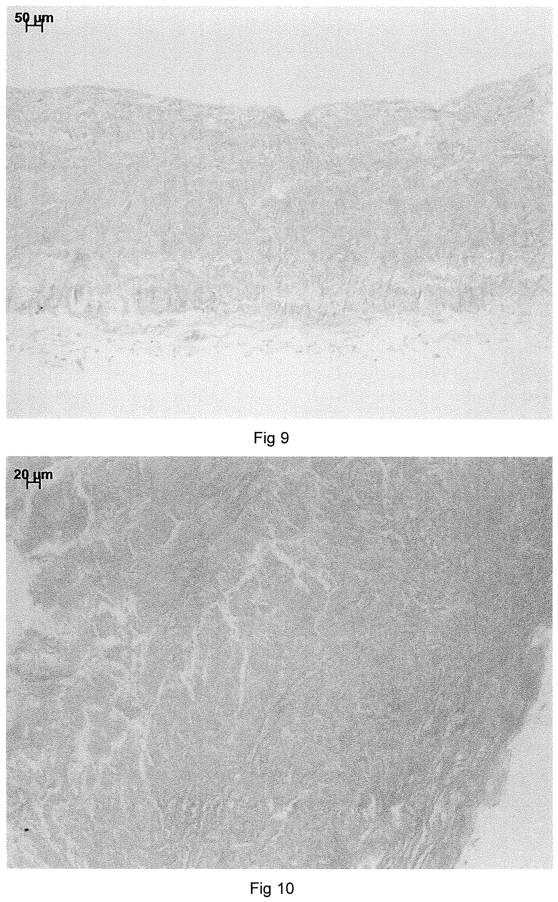

Figure 7 is a light microscopy photograph of pericardial tissue treated with the single deoxycholic acid solution. Figure 8 is a light microscopy photograph of pericardial tissue treated with the double deoxycholic acid solution. Figure 9 is a light microscopy photograph of pericardial tissue treated with the triple deoxycholic acid solution of the invention. From Figure 7, it can be seen that treatment with a single solution containing deoxycholic acid, one a large part of the tissue is free of cells, however not completely. Figure 8, which shows the result of treatment with the double deoxycholic acid solution shows an improvement, with additional reduction of cells in the tissue. However, as shown in Figure 9, it is only the triple deoxycholic acid solution of the invention where there are no cells available any longer, and thus achieves complete decellularization.

Figure 10 is a light microscopy photograph of myocardium tissue treated with the triple deoxycholic acid solution of the invention. As shown in Figure 10, the triple deoxycholic acid solution of the invention, and thus achieves complete decellularization.

## Claims

1. A method of detoxification and stabilization of implantable or transplantable biological material of human or animal origin, the method including the following successive steps:
1) treatment of the material with an antibiotic solution;
2) treatment of the material in a solution containing a secondary bile acid surfactant;
3) treatment of the material in a solution to remove the secondary bile acid surfactant; and
4) treatment of the material in a primary alcohol wherein optionally the primary alcohol is ethanol;
wherein the solution at Step 2) containing a secondary bile acid surfactant contains:
• the secondary bile acid surfactant at a concentration of 0.1 - 2% v/v;
• an anionic surfactant at a concentration of 1-3% v/v, and
• Triton-x100 surfactant at a concentration of 0.1 - 3% v/v.

2. The method claimed in claim 1, wherein the secondary bile acid surfactant is deoxycholic acid or ursodeoxycholic acid.

3. The method claimed in any one of the preceding claims, wherein the anionic surfactant is a sulfate, wherein optionally the sulfate is sodium dodecyl sulfate, ammonium dodecyl sulfate or potassium lauryl sulfate.

4. The method claimed in claim 1, wherein the solution containing the secondary bile acid surfactant contains:
MH:aw
the secondary bile acid surfactant at a concentration of 0.5% v/v;
the anionic surfactant at a concentration of 2% v/v; and
the Triton-x100 surfactant at a concentration 0.5% v/v.

5. The method claimed in any one of the preceding claims, wherein the antibiotic solution contains at least one of the following antibiotics:
Amphotericin B, preferably in an amount of 5-100µg/ml, more preferably in an amount of 5-15µg/ml, most preferably 10µg/ml,
Ciprofloxacin, preferably in an amount of 5 - 200µg/ml, more preferably in an amount of 45 - 55µg/ml, most preferably 50µg/ml,
Cefuroxin, preferably in an amount of 20-1500µg/ml, more preferably in an amount of 740-760µg/ml, most preferably 750µg/ml,
Penicillin, preferably in an amount of 20 - 1000µg/ml, more preferably in an amount of 180 - 220µg/ml, most preferably 200µg/ml,
Strepotomycin, preferably in an amount of 20 - 1000µg/ml, more preferably in an amount of 180 - 220µg/ml, most preferably 200µg/ml.

6. The method claimed in any one of the preceding claims, wherein the antibiotic solution contains no Ca or Mg.

7. The method claimed in any one of the preceding claims, wherein the solution to remove the deoxycholic acid or ursodeoxycholic acid contains a lipopeptide antimicrobial agent wherein optionally the lipopeptide antimicrobial agent is Fengycin or Iturin A.

8. The method claimed in any one of the preceding claims wherein, after step 4), the material is introduced to a storage solution containing antibiotic(s), wherein optionally the storage solution contains one or all antibiotic(s) selected from the list consisting of Amphotericin B, Penicillin, Streptomycin.

9. The method claimed in claim 8, wherein the storage solution contains the following antibiotics:
Amphotericin B, in an amount of 5-15µg/ml, preferably 10µg/ml,
Penicillin, in an amount of 80 - 120µg/ml, preferably 100µg/ml,
Streptomycin, in an amount of 80 - 120µg/ml, preferably 100µg/ml.

10. A solution containing a secondary bile acid surfactant for use in a method of detoxification and stabilization of implantable or transplantable biological material of human or animal origin, the solution containing:
• the secondary bile acid surfactant at a concentration of 0.1 - 2% v/v;
• an anionic surfactant at a concentration of 1-3% v/v, and
• Triton-x100 surfactant at a concentration of 0.1 - 3% v/v.

11. The solution claimed in claim 10, the secondary bile acid surfactant is deoxycholic acid or ursodeoxycholic acid.

12. The solution claimed in any one of claims 10 to 11, wherein the anionic surfactant is a sulfate.

13. The solution claimed in claim 12, wherein the sulfate is sodium dodecyl sulfate, ammonium dodecyl sulfate or potassium lauryl sulfate.

14. The solution claimed in claim 13, wherein the solution containing a secondary bile acid surfactant contains:
the secondary bile acid surfactant at a concentration of 0.5% v/v;
the anionic surfactant at a concentration of 2% v/v; and
the Triton-x100 surfactant at a concentration 0.5% v/v.

## Patentansprüche

1. Verfahren zur Detoxifikation und Stabilisierung von implantierbarem oder transplantierbarem biologischen Material menschlichen oder tierischen Ursprungs, umfassend die folgenden aufeinanderfolgenden Schritte:
1) Behandlung des Materials mit einer antibiotischen Lösung;
2) Behandlung des Materials in einer Lösung enthaltend ein sekundäres Gallensäuretensid;
3) Behandlung des Materials in einer Lösung, um das sekundäre Gallensäuretensid zu entfernen;
4) Behandlung des Materials in einem Primäralkohol, optional wobei der Primäralkohol Ethanol ist;
wobei die Lösung in Schritt 2) enthaltend ein sekundäres Gallensäuretensid, enthält:
- das sekundäre Gallensäuretensid in einer Konzentration von 0,1-2% v/v;
- ein anionisches Tensid in einer Konzentration von 1-3% v/v, und
- Triton-x 100 Tensid in einer Konzentration von 0,1-3% v/v.

2. Verfahren nach Anspruch 1, wobei das sekundäre Gallensäuretensid Desoxycholsäure oder Ursodeoxycholsäure ist.

3. Verfahren nach einem der vorhergehenden Ansprüche, wobei das anionische Tensid ein Sulfat ist, optional wobei das Sulfat Natruimdodecylsulfat, Ammoniumdodecylsulfat oder Kaliumlaurylsulfat ist.

4. Verfahren nach Anspruch 1, wobei die Lösung enthaltend das sekundäre Gallensäuretensid enthält:
das sekundäre Gallensäuretensid in einer Konzentration von 0,5% v/v;
das anionische Tensid in einer Konzentration von 2% v/v, und
das Triton-x 100 Tensid in einer Konzentration von 0,5% v/v.

5. Verfahren nach einem der vorhergehenden Ansprüche, wobei die antibiotische Lösung mindestens eines der folgenden Antibiotika enthält:
Amphotericin B, bevorzugt in einer Menge von 5-100 µg/ml, bevorzugter in einer Menge von 5-15 µg/ml, am meisten bevorzugt 10 µg/ml,
Ciprofloxacin, bevorzugt in einer Menge von 5-200 µg/ml, bevorzugter in einer Menge von 45-55 µg/ml, am meisten bevorzugt 50 µg/ml,
Cefuroxin, bevorzugt in einer Menge von 20-1500 µg/ml, bevorzugter in einer Menge von 740-760 µg/ml, am meisten bevorzugt 750 µg/ml,
Penicillin, bevorzugt in einer Menge von 20-1000 µg/ml, bevorzugter in einer Menge von 180-220 µg/ml, am meisten bevorzugt 200 µg/ml,
Streptomycin, bevorzugt in einer Menge von 20-1000 µg/ml, bevorzugter in einer Menge von 180-220 µg/ml, am meisten bevorzugt 200 µg/ml.

6. Verfahren nach einem der vorhergehenden Ansprüche, wobei die antibiotische Lösung kein Ca oder Mg enthält.

7. Verfahren nach einem der vorhergehenden Ansprüche, wobei die Lösung, um die Desoxycholinsäure oder Ursodesoxycholinsäure zu entfernen, ein Lipopeptidantimikrobielles Mittel enthält, optional wobei das Lipopeptid-antimikrobielle Mittel Fengycin oder Iturin A ist.

8. Verfahren nach einem der vorhergehenden Ansprüche, wobei, nach Schritt 4), das Material in eine Aufbewahrungslösung gegeben wird, enthaltend ein Antibiotikum/Antibiotika, optional wobei die Aufbewahrungslösung eines oder alle der Antibiotika enthält, die ausgewählt sind aus der Liste bestehend aus Amphotericin, Penicillin, Streptomycin.

9. Verfahren nach Anspruch 8, wobei die Aufbewahrungslösung die folgenden Antibiotika enthält:
Amphotericin B, in einer Menge von 5-15 µg/ml, bevorzugt 10 µg/ml,
Penicillin, in einer Menge von 80-120 µg/ml, bevorzugt 100 µg/ml,
Streptomycin, in einer Menge von 80-120 µg/ml, bevorzugt 100 µg/ml.

10. Lösung enthaltend ein sekundäres Gallensäuretensid zur Verwendung in einem Verfahren zur Detoxifizierung und Stabilisierung eines implantierbaren oder transplantierbaren biologischen Materials menschlichen oder tierischen Ursprungs, wobei die Lösung enthält:
- das sekundäre Gallensäuretensid in einer Konzentration von 0,1-2% v/v;
- ein anionisches Tensid in einer Konzentration von 1-3% v/v, und
- Triton-x 100 Tensid in einer Konzentration von 0,1-3% v/v.

11. Lösung nach Anspruch 10, wobei das sekundäre Gallensäuretensid Desoxycholsäure oder Ursodeoxycholsäure ist.

12. Lösung nach einem der Ansprüche 10 bis 11, wobei das anionische Tensid ein Sulfat ist.

13. Lösung nach Anspruch 12, wobei das Sulfat Natruimdodecylsulfat, Ammoniumdodecylsulfat oder Kaliumlaurylsulfat ist.

14. Lösung nach Anspruch 13, wobei die Lösung enthaltend ein sekundäres Gallensäuretensid enthält:
das sekundäre Gallensäuretensid in einer Konzentration von 0,5% v/v;
das anionische Tensid in einer Konzentration von 2% v/v, und
das Triton-x 100 Tensid in einer Konzentration von 0,5% v/v.

## Revendications

1. Procédé de détoxication et de stabilisation de matériau biologique implantable ou transplantable d'origine humaine ou animale, le procédé incluant les étapes successives suivantes :
1) traitement du matériau avec une solution antibiotique ;
2) traitement du matériau dans une solution contenant un agent tensioactif d'acide biliaire secondaire ;
3) traitement du matériau dans une solution permettant d'enlever l'agent tensioactif d'acide biliaire secondaire ; et
4) traitement du matériau dans un alcool primaire, sachant que facultativement l'alcool primaire est de l'éthanol ;
sachant que la solution à l'étape 2) contenant un agent tensioactif d'acide biliaire secondaire contient :
- l'agent tensioactif d'acide biliaire secondaire à une concentration de 0,1 à 2 % v/v ;
- un agent tensioactif anionique à une concentration de 1 à 3 % v/v, et
- un agent tensioactif Triton-x 100 à une concentration de 0,1 à 3 % v/v.

2. Le procédé revendiqué dans la revendication 1, sachant que l'agent tensioactif d'acide biliaire secondaire est de l'acide désoxycholique ou de l'acide ursodésoxycholique.

3. Le procédé revendiqué dans l'une quelconque des revendications précédentes, sachant que l'agent tensioactif anionique est un sulfate, sachant que facultativement le sulfate est du dodécylsulfate de sodium, du dodécylsulfate d'ammonium ou du laurylsulfate de potassium.

4. Le procédé revendiqué dans la revendication 1, sachant que la solution contenant l'agent tensioactif d'acide biliaire secondaire contient :
l'agent tensioactif d'acide biliaire secondaire à une concentration de 0,5 % v/v ;
l'agent tensioactif anionique à une concentration de 2 % v/v ; et
l'agent tensioactif Triton-x100 à une concentration de 0,5 % v/v.

5. Le procédé revendiqué dans l'une quelconque des revendications précédentes, sachant que la solution antibiotique contient au moins l'un des antibiotiques suivants :
de l'amphotéricine B, de préférence dans une quantité de 5 à 100 µg/ml, de façon plus préférentielle dans une quantité de 5 à 15 µg/ml, de façon la plus préférentielle de 10 µg/ml,
de la ciprofloxacine, de préférence dans une quantité de 5 à 200 µg/ml, de façon plus préférentielle dans une quantité de 45 à 55 µg/ml, de façon la plus préférentielle de 50 µg/ml,
de la céfuroxine, de préférence dans une quantité de 20 à 1500 µg/ml, de façon plus préférentielle dans une quantité de 740 à 760 µg/ml, de façon la plus préférentielle de 750 µg/ml,
de la pénicilline, de préférence dans une quantité de 20 à 1000 µg/ml, de façon plus préférentielle dans une quantité de 180 à 220 µg/ml, de façon la plus préférentielle de 200 µg/ml,
de la streptomycine, de préférence dans une quantité de 20 à 1000 µg/ml, de façon plus préférentielle dans une quantité de 180 à 220 µg/ml, de façon la plus préférentielle de 200 µg/ml.

6. Le procédé revendiqué dans l'une quelconque des revendications précédentes, sachant que la solution antibiotique ne contient pas de Ca ou de Mg.

7. Le procédé revendiqué dans l'une quelconque des revendications précédentes, sachant que la solution permettant d'enlever l'acide désoxycholique ou l'acide ursodésoxycholique contient un agent antimicrobien lipopeptide, sachant que facultativement l'agent antimicrobien lipopeptide est de la fengycine ou de l'iturine A.

8. Le procédé revendiqué dans l'une quelconque des revendications précédentes, sachant que, après l'étape 4), le matériau est introduit dans une solution de stockage contenant un ou des antibiotiques, sachant que facultativement la solution de stockage contient un ou la totalité des antibiotiques sélectionnés dans la liste constituée par l'amphotéricine B, la pénicilline, la streptomycine.

9. Le procédé revendiqué dans la revendication 8, sachant que la solution de stockage contient les antibiotiques suivants :
de l'amphotéricine B, dans une quantité de 5 à 15 µg/ml, de préférence de 10 µg/ml,
de la pénicilline, dans une quantité de 80 à 120 µg/ml, de préférence de 100 µg/ml,
de la streptomycine, dans une quantité de 80 à 120 µg/ml, de préférence de 100 µg/ml.

10. Solution contenant un agent tensioactif d'acide biliaire secondaire destinée à être utilisée dans un procédé de détoxication et de stabilisation de matériau biologique implantable ou transplantable d'origine humaine ou animale, la solution contenant :
- l'agent tensioactif d'acide biliaire secondaire à une concentration de 0,1 à 2 % v/v ;
- un agent tensioactif anionique à une concentration de 1 à 3 % v/v, et
- un agent tensioactif Triton-x100 à une concentration de 0,1 à 3 % v/v.

11. La solution revendiquée dans la revendication 10, sachant que l'agent tensioactif d'acide biliaire secondaire est de l'acide désoxycholique ou de l'acide ursodésoxycholique.

12. La solution revendiquée dans l'une quelconque des revendications 10 à 11, sachant que l'agent tensioactif anionique est un sulfate.

13. La solution revendiquée dans la revendication 12, sachant que le sulfate est du dodécylsulfate de sodium, du dodécylsulfate d'ammonium ou du laurylsulfate de potassium.

14. La solution revendiquée dans la revendication 13, sachant que la solution contenant un agent tensioactif d'acide biliaire secondaire contient :
l'agent tensioactif d'acide biliaire secondaire à une concentration de 0,5 % v/v ;
l'agent tensioactif anionique à une concentration de 2 % v/v ; et
l'agent tensioactif Triton-x100 à une concentration de 0,5 % v/v.
